Europäisches Patentamt

European Patent Office

Office européen des brevets

(19)

(11) **EP 1 514 923 A1**

(12) **EUROPEAN PATENT APPLICATION**
published in accordance with Art. 158(3) EPC

(43) Date of publication:
    **16.03.2005 Bulletin 2005/11**

(51) Int Cl.⁷: **C12N 9/44**, C12N 9/16,
     C12P 19/04, C08B 37/00
     // C12N15:12

(21) Application number: **03733522.1**

(22) Date of filing: **20.06.2003**

(86) International application number:
    **PCT/JP2003/007838**

(87) International publication number:
    **WO 2004/001031 (31.12.2003 Gazette 2004/01)**

(84) Designated Contracting States:
    **AT BE BG CH CY CZ DE DK EE ES FI FR GB GR
    HU IE IT LI LU MC NL PT RO SE SI SK TR**
    Designated Extension States:
    **AL LT LV MK**

(30) Priority: **20.06.2002 JP 2002180490
              20.08.2002 JP 2002239843**

(71) Applicant: **TAKARA BIO INC.
    Otsu-shi, Shiga 520-2193 (JP)**

(72) Inventors:
    • **SAKAI, Takeshi
      Kusatsu-shi, Shiga 525-0072 (JP)**
    • **ISHIZUKA, Kumiko
      Hirosaki-shi, Aomori 036-8171 (JP)**
    • **KATO, Ikunoshin
      Uji-shi, Kyoto 611-0028 (JP)**

(74) Representative: **Grund, Martin, Dr. et al
    Dr. Volker Vossius,
    Patentanwaltskanzlei,
    Geibelstrasse 6
    81679 München (DE)**

(54) **ENZYME FOR DECOMPOSITION OF SULFATED FUCAN DERIVED FROM SEA CUCUMBER**

(57)    Sulfated fucan lyase capable of decomposing sulfated fucan derived from sea cucumber; a process for producing the enzyme; a low molecular compound obtained by causing the enzyme to act on sulfated fucan; a process for producing the same; and an activating factor for sulfated fucan lyase.

EP 1 514 923 A1

## SEQUENCE LISTING

<110> TAKARA BIO INC.

<120> Enzyme for decomposition of sulfated fucan derived from sea cucumber

<130> 38-82

<150> JP 2002-180490
<151> 2002-06-20

<150> JP 2002-239843
<151> 2002-08-20

<160> 1

<210> 1
<211> 1521
<212> DNA
<213> Fucoidanobacter marinus SI-0098

<400> 1

```
agagtttgat cctggctcag aatgaacgct ggcggcgtgg ttcagacatg caagtcgaac    60
gggattgtct agttagcttg ctaattagac atgagagtgg cgaacgggtg cgtaacacgt   120
aaagaaccta cccttatgtg ggggatagct caccgaangg tgaattaata ccgcatgtgg   180
tctctcttca catgaagagt acactnaagc tggggacctt cgggcctggc gcatagggag   240
ggctttgcgg cctatcagct tgttggtgag gtaacggctc accaaggcaa agacnggtag   300
ctggtctgag aagatgatca gccacactgg aacttagaca cggtccagac acctacgggt   360
ggcagcagtt tcgaatcttt cacaatgggc gaaagcctga tggagcaacg ccgcgtgggg   420
```

gatgaaggcc ttcgggttgt aaacccctgt caccaaggat aaaacgtaat ctattaatac   480

taggttgcct gatgtaactt ggagaggaag gagtggctaa ctctgtgcca gcagccgcgg   540

taatacagag actccaagcg ttattcggat tcactgggcg taaagggagc gcaggcggcc   600

agatgtgtca gaggtgaaat accgcagctt aactgtagaa ctgcctttga aactatctgg   660

ctagagtatc ggagaggtaa gcggaattcc aggtgtagca gtgaaatgcg tagatatctg   720

gaggaacacc aatggcgaag gcagcttact ggacgattac tgacgctcag gctcgaaagc   780

atggggagcg aaagggatta gatacccctg tagtccatgc cgtaaacgtt gttcactagg   840

tatcgggaca ttcgaccgtc tcggtgctca agctaacgcg ataagtgaac cgcctgagga   900

ctacggccgc aaggctaaaa ctcaaaggaa ttgacgggag cctgcacaag cggtggagca   960

tgtggcttaa ttcgatgcaa cgcgaagaac cttacctagg cttgacatgc agtggaccgg   1020

ggcagagatg ccctttctct tcggagccgc tgcacaggtg ctgcatggct gtcgtcagct   1080

cgtgtcgtga gatgtttggt taagtccagc aacgagcgca acccctgcca ctagttgcca   1140

gcattaagtt ggggactcta gtgggacaaa ctctctctga gagtgggaag gtggggacga   1200

cgtcaagtca gtatggccct tacgtctagg gctgcacacg tgctacaatg cccggtacag   1260

agggacgcga taccgcgagg tggagcaaat ccttaaagcc gggcccagtt cagattggag   1320

tctgcaactc gactccatga agttggaatc gctagtaatg gcgcatcagc tatggcgccg   1380

tgaatacgtt cccaggcctt gtacacaccg cccgtcacgt tatggaagcc ggttttgccc   1440

gaagtatgtt agctaacccg caagggaggc gatgtcctaa ggtgaggctg gtaactggaa   1500

cgaagtcgta acaaggtagc c     1521

## Description

Technical Field

The present invention relates to an enzyme that degrades a sulfated fucan which is useful in a field of glycotechnology, a method for producing the enzyme and various factors that activate the enzyme, as well as a sulfated fucan oligosaccharide which is useful as a reagent for glycotechnology and a method for producing the same.

Background Art

Several kinds of sulfated polysaccharides are contained in a sea cucumber. They include a sulfated fucan which is a polysaccharide whose main component is sulfated fucose. An activity of suppressing infection with AIDS virus and an anticoagulant activity of a sulfated fucan derived from a sea cucumber have been reported. If a sulfated fucan derived from a sea cucumber is to be developed as a pharmaceutical, it is necessary to determine its structure. The structure of a sulfated fucan derived from a sea cucumber has been studied although only average values for the structure have been determined at present. It would be very advantageous if an enzyme that degrades a sulfated fucan is used to determine the structure of the sulfated fucan. However, no enzyme that degrades a sulfated fucan derived from a sea cucumber is commercially available. Sulfated fucans are also contained in various brown algae, and their structures may vary depending on the algae from which they are derived in many cases. For example, sulfated fucans extracted from *Fucus evanescens, Laminaria japonica* and *Nemacystus decipiens* have structures different from each other. Several enzymes that degrade a sulfated fucan derived from brown algae have reported (WO 96/34004, WO 97/26896, WO 99/11797, WO 99/41288 and EP 1306389), although none of them cannot be used to degrade a sulfated fucan derived from a sea cucumber. Thus, it is considered that the structure of the sea cucumber-derived sulfated fucan is different from the structures of the brown algae-derived sulfated fucans.

As described above, an enzyme that degrades a sulfated fucan derived from a sea cucumber, and an enzymatically produced and structurally homogeneous sulfated fucan oligosaccharide have been desired.

Objects of Invention

Thus, the main object of the present invention is to provide a sulfated fucan-degrading enzyme that degrades a sulfated fucan derived from a sea cucumber and a method for producing the enzyme, as well as a smaller molecule that is obtainable by allowing the enzyme to act on a sulfated fucan and a method for producing the same. A further object of the present invention is to provide a factor that activates a sulfated fucan-degrading enzyme.

Summary of Invention

The present inventors have found that a bacterial strain belonging to the genus *Fucoidanobacter, Fucoidanobacter marinus* SI-0098, produces a novel sulfated fucan-degrading enzyme, and found a method for producing the enzyme. The present inventors have also found that a novel sulfated fucan oligosaccharide can be produced from a sulfated fucan derived from a sea cucumber utilizing the enzyme. Thus, the present invention has been completed.

The first aspect of the present invention relates to an endo-type α-L-fucosidase which is obtainable from a culture of a bacterium of the genus *Fucoidanobacter.*

According to the first aspect, the enzyme can be collected from a culture of a bacterium of the genus *Fucoidanobacter* capable of producing the endo-type α-L-fucosidase.

The second aspect of the present invention relates to a sulfated fucan sulfatase which is obtainable from a culture of a bacterium of the genus *Fucoidanobacter.*

According to the second aspect, the enzyme can be collected from a culture of a bacterium of the genus *Fucoidanobacter* capable of producing the sulfated fucan sulfatase.

The third aspect of the present invention relates to a sulfated fucan oligosaccharide which is obtainable by allowing the endo-type α-L-fucosidase of the first aspect to act on a fraction of a sulfated fucan derived from a sea cucumber.

The sulfated fucan oligosaccharide of the third aspect can be produced according to a method for producing a sulfated fucan oligosaccharide comprising allowing the endo-type α-L-fucosidase of the first aspect to act on a fraction of a sulfated fucan derived from a sea cucumber. The method may be carried out in the presence of sodium chloride.

The fourth aspect of the present invention relates to a sulfated fucan oligosaccharide which is obtainable by allowing the endo-type α-L-fucosidase of the first aspect and the sulfated fucan sulfatase of the second aspect to act on a fraction of a sulfated fucan derived from a sea cucumber.

The sulfated fucan oligosaccharide of the fourth aspect can be produced according to a method for producing a sulfated fucan oligosaccharide comprising allowing the endo-type α-L-fucosidase of the first aspect and the sulfated fucan sulfatase of the second aspect to act on a fraction of a sulfated fucan derived from a sea cucumber. The method may be carried out in the presence of sodium chloride and/or calcium chloride.

Brief Description of Drawings

Figure 1: a graph that illustrates the relationship between the pH and the relative activity (%) of the endo-type α-L-fucosidase according to the present invention.
Figure 2: a graph that illustrates the relationship between the temperature (°C) and the relative activity (%) of the endo-type α-L-fucosidase according to the present invention.
Figure 3: a graph that illustrates the relationship between the salt concentration (mM) and the relative activity (%) of the endo-type α-L-fucosidase according to the present invention.
Figure 4: a graph that illustrates the relationship between the pH and the relative activity (%) of the sulfated fucan sulfatase according to the present invention.
Figure 5: a graph that illustrates the relationship between the temperature (°C) and the relative activity (%) of the sulfated fucan sulfatase according to the present invention.
Figure 6: a graph that illustrates the relationship between the salt concentration (mM) and the relative activity (%) of the sulfated fucan sulfatase according to the present invention.
Figure 7: a figure that illustrates the elution pattern of gel filtration of sulfated fucan oligosaccharides obtained by degrading a sulfated fucan using the endo-type α-L-fucosidase according to the present invention.
Figure 8: a figure that illustrates the results of HPLC analysis of sulfated fucan oligosaccharides obtained by degrading a sulfated fucan using the endo-type α-L-fucosidase and the sulfated fucan sulfatase according to the present invention.

Detailed Description of the Invention

Hereinafter, the present invention will be described in detail.
Although it is not intended to limit the present invention, for example, a sulfated fucan derived from *Apostichopus japonicus* can be used as a sulfated fucan derived from a sea cucumber according to the present invention. *Apostichopus japonicus* is preferable as a raw material because a high efficiency of producing the sulfated fucan oligosaccharide of the present invention can be achieved with it.
The endo-type α-L-fucosidase of the present invention is an enzyme that acts on a sulfated fucan derived from a sea cucumber to generate an oligosaccharide having fucose at the reducing end.
The sulfated fucan sulfatase of the present invention is an enzyme that releases sulfuric acid upon its action on an oligosaccharide generated by allowing the endo-type α-L-fucosidase of the present invention on a sulfated fucan derived from a sea cucumber or *Apostichopus japonicus.*
The sulfated fucan oligosaccharide of the present invention is an oligosaccharide that is obtainable by allowing the endo-type α-L-fucosidase of the present invention alone or together with the sulfated fucan sulfatase of the present invention to act on a sulfated fucan derived from a sea cucumber. It has fucose at the reducing end.
A sulfated polysaccharide fraction is first extracted by soaking a sea cucumber in an aqueous solvent in order to produce a sulfated fucan derived from a sea cucumber used according to the present invention. In this case, it is preferable to obtain the fraction at pH 4-9 at a temperature of 100°C or below in order to prevent the conversion of the sulfated fucan into smaller molecules. Furthermore, amino acids, small molecule pigments or the like in the sulfated polysaccharide fraction can be efficiently removed by ultrafiltration. Active carbon treatment or the like is effective for the removal of hydrophobic substances.
A sulfated polysaccharide fraction from a sea cucumber can be obtained as described above. This fraction can be used as a sulfated fucan fraction, for example, as a substrate for measuring an activity of the endo-type α-L-fucosidase or the sulfated fucan sulfatase of the present invention, or as a substrate for producing the sulfated fucan oligosaccharide of the present invention. A more highly pure sulfated fucan can be obtained by separating the fraction using an anion exchange column. Both the sulfated polysaccharide fraction and the sulfated fucan purified using an anion exchange column can be used as substrates for measuring an activity of the endo-type α-L-fucosidase or the sulfated fucan sulfatase of the present invention, or as substrates for producing the sulfated fucan oligosaccharide of the present invention.
There is no specific limitation concerning the bacterium used for producing the endo-type α-L-fucosidase or the sulfated fucan sulfatase of the present invention as long as it is a bacterium that produces the enzyme. For example, *Fucoidanobacter* marines SI-0098 may be used. This bacterial strain is a bacterium isolated by the present inventors.

This strain was indicated as *Fucoidanobacter marinus* SI-0098 and deposited at International Patent Organism Depositary, National Institute of Advanced Science and Technology (AIST Tsukuba Central 6, 1-1, Higashi 1-chome, Tsukuba, Ibaraki 305-8566, Japan) under accession number FERM P-14873 on March 29, 1995 (date of original deposit), and deposited at International Patent Organism Depositary, National Institute of Advanced Science and Technology under Budapest Treaty under accession number FERM BP-5403 on February 15, 1996 (date of request for transmission to international depositary authority).

The present inventors determined the nucleotide sequence of 16S rRNA-encoding DNA of this strain, examined its homology to those of known bacteria, and confirmed that this strain is a bacterium belonging to a new genus with the closest relation to Verrucomicrobia.

The sequence of 16S rRNA-encoding DNA of this strain is shown in SEQ ID N0:1. The endo-type $\alpha$-L-fucosidases, or the sulfated fucan sulfatases, of the present invention include an endo-type $\alpha$-L-fucosidase, or a sulfated fucan sulfatase, obtained from a bacterium that is determined to belong to the genus to which *Fucoidanobacter marinus* SI-0098 belongs based on the sequence of 16S rRNA-encoding DNA.

Any nutrient source can be added to a medium for culturing a bacterium that produces the endo-type $\alpha$-L-fucosidase or the sulfated fucan sulfatase of the present invention as long as it is utilized by the microorganism to produce the enzyme in the presence of the nutrient source. For example, a sulfated fucan, a sea cucumber, a dried sea cucumber, an alga, alginic acid, laminaran, fucose, glucose, mannitol, glycerol, saccharose, maltose, starch or the like can be utilized as a carbon source. Yeast extract, peptone, casamino acid, corn steep liquor, meat extract, defatted soybean, ammonium sulfate, ammonium chloride, urea, uric acid or the like is suitable as a nitrogen source. In addition, a chloride, a phosphate or a sulfate of sodium, potassium, magnesium, calcium, zinc or the like may be added. In general, a microorganism isolated from seawater can be very readily grown in seawater or commercially available artificial seawater.

The culture conditions are determined such that the productivity of the endo-type $\alpha$-L-fucosidase or the sulfated fucan sulfatase of the present invention becomes maximal depending on the microorganism to be used, the composition of the medium and the like. In general, the maximal productivity of the endo-type $\alpha$-L-fucosidase or the sulfated fucan sulfatase of the present invention is achieved by culturing with aeration and stirring at a culture temperature of 15 to 30°C at medium pH of 5 to 9 for 5 to 72 hours. The endo-type $\alpha$-L-fucosidase or the sulfated fucan sulfatase of the present invention can be obtained from cells and a culture supernatant separated from each other by centrifugation after culturing.

A cell-free extract is obtained by culturing *Fucoidanobacter marinus* SI-0098 in an appropriate medium, collecting the cells, and disrupting the cells using a conventional means of cell disruption (e.g., sonication). The endo-type $\alpha$-L-fucosidase or the sulfated fucan sulfatase of the present invention can be purified from the extract using a conventional means of purification. The endo-type $\alpha$-L-fucosidase or the sulfated fucan sulfatase of the present invention can be obtained in a purified form using, for example, salting out, ion exchange column chromatography, hydrophobic column chromatography, gel filtration or the like. Furthermore, a means of purification similar to that used for the purification of the intracellular enzyme can be used to purify the endo-type $\alpha$-L-fucosidase or the sulfated fucan sulfatase of the present invention from the culture supernatant which also contains the enzyme.

The physical and chemical properties of the endo-type $\alpha$-L-fucosidase of the present invention are as follows:

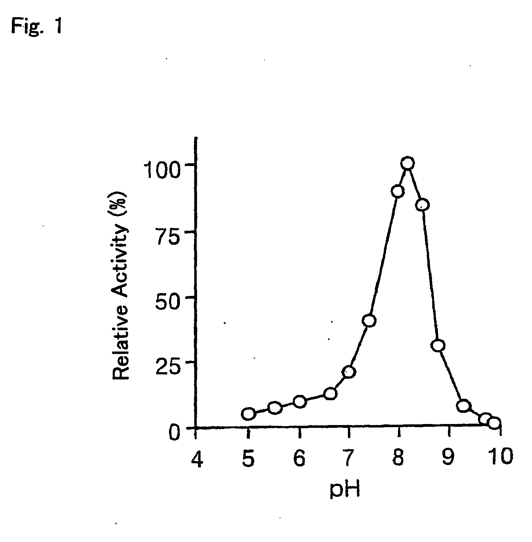

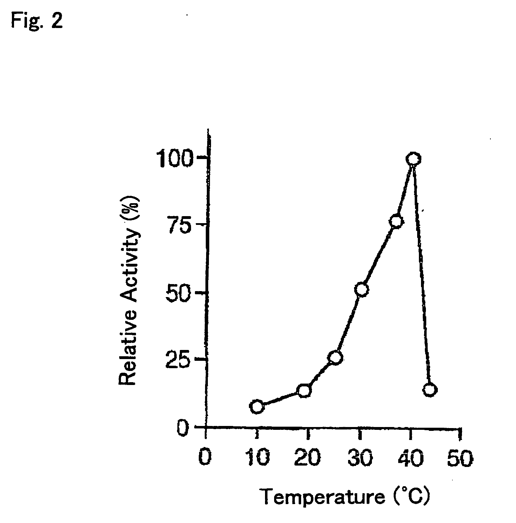

(I) acting on a sulfated fucan derived from a sea cucumber to hydrolyze an $\alpha$-L-fucosyl bond;
(II) having an optimal pH of about 7 to 9 (Figure 1, a graph illustrating the relationship between the reaction pH and the relative activity of the enzyme, in which the longitudinal axis represents the relative activity (%) and the horizontal axis represents the pH); and
(III) having an optimal temperature of about 25 to 45°C (Figure 2, a graph which illustrates the relationship between the reaction temperature and the relative activity of the enzyme, in which the longitudinal axis represents the relative activity (%) and the horizontal axis represents the temperature (°C)).

The endo-type $\alpha$-L-fucosidase of the present invention can be identified by measuring an activity of degrading a sulfated fucan. A supernatant obtained by centrifugation of a culture of a producer strain, a cell-free extract, or an enzyme solution obtained after purification using various column chromatographies may be used for the identification.

The endo-type $\alpha$-L-fucosidase of the present invention is activated in the presence of sodium chloride.

Any material containing sodium chloride such as sodium chloride as a reagent, table salt, seawater or artificial seawater may serve as sodium chloride. The concentration of sodium chloride to be added to a reaction mixture for the endo-type $\alpha$-L-fucosidase of the present invention may range from about 1' mM to about 1 M, preferably from about 5 mM to about 900 mM.

The physical and chemical properties of the sulfated fucan sulfatase of the present invention are as follows:

(I) acting on a sulfated fucan derived from a sea cucumber to release sulfuric acid and promote conversion of the sulfated fucan derived from a sea cucumber into smaller molecules in the presence of the endo-type $\alpha$-L-fucosidase of the present invention as compared with the conversion achieved by allowing the endo-type $\alpha$-L-fucosidase of the present invention to act alone;

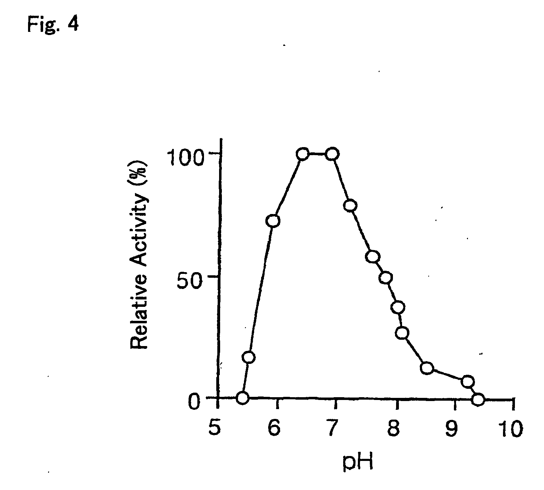

(II) having an optimal pH of about 6 to 8 (Figure 4, a graph illustrating the relationship between the reaction pH and the relative activity of the enzyme, in which the longitudinal axis represents the relative activity (%) and the horizontal axis represents the pH); and

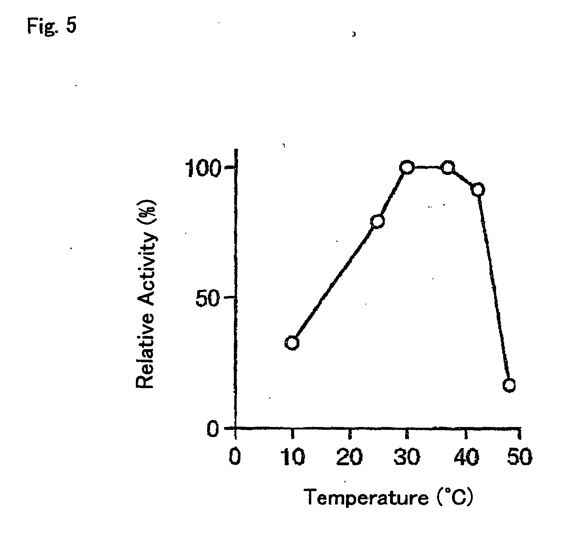

(III) having an optimal temperature of about 20 to 45°C (Figure 5, a graph which illustrates the relationship between the reaction temperature and the relative activity of the enzyme, in which the longitudinal axis represents the relative activity (%) and the horizontal axis represents the temperature (°C)).

The sulfated fucan sulfatase of the present invention can be identified by measuring released sulfuric acid. A supernatant obtained by centrifugation of a culture of a producer strain, a cell-free extract, or an enzyme solution obtained after purification using various column chromatographies may be used for the identification.

The sulfated fucan sulfatase of the present invention is activated in the presence of sodium chloride and/or a calcium ion.

Regarding sodium chloride, any material containing sodium chloride such as sodium chloride as a reagent, table salt, seawater or artificial seawater may serve as sodium chloride. The concentration of sodium chloride to be added to a reaction mixture for the sulfated fucan sulfatase of the present invention may range from about 1 mM to about 2. M, preferably from about 5 mM to about 1 M.

Regarding the calcium ion, any material containing a calcium ion such as calcium chloride or calcium acetate as reagents may serve as a calcium ion. The concentration of a calcium ion to be added to a reaction mixture for the sulfated fucan sulfatase of the present invention may range from about 0.1 mM to about 1 M, preferably from about 1 mM to about 500 mM.

*Fucoidanobacter marinus* SI-0098 is a microorganism that utilizes a sulfated fucan, and produces the endo-type $\alpha$-L-fucosidase and the sulfated fucan sulfatase of the present invention inside or outside the cell to degrade the sulfated fucan.

The sulfated fucan oligosaccharide of the present invention is an oligosaccharide that is obtainable by allowing the endo-type $\alpha$-L-fucosidase of the present invention alone or together with the sulfated fucan sulfatase of the present invention to act on a sulfated fucan derived from a sea cucumber or a sulfated fucan-containing material. It has fucose at the reducing end. For example, a partially purified preparation of a sulfated fucan, a sulfated polysaccharide fraction derived from a sea cucumber, an extract of a sea cucumber obtained using an aqueous solvent, a dried sea cucumber or a raw sea cucumber can be preferably used as a sulfated fucan-containing material.

For preparing the sulfated fucan oligosaccharide of the present invention, a sulfated fucan or a sulfated fucan-containing material may be dissolved according to a conventional method. The sulfated fucan or the sulfated fucan-containing material may be dissolved in the solution at the maximal concentration. However, the concentration is usually selected taking its operationality and the amount of the endo-type $\alpha$-L-fucosidase or the sulfated fucan sulfatase of the present invention to be used in a reaction into consideration. The solvent for the sulfated fucan may be selected from water, buffers and the like depending on the objects. There is no specific limitation concerning the reaction conditions as long as the enzyme to be used exhibits its activity. Usually, the pH of the solution is nearly neutral. The enzymatic reaction is usually carried out at about 30°C. The molecular weight of the sulfated fucan oligosaccharide can be controlled by adjusting the ratio or the amounts of the endo-type $\alpha$-L-fucosidase and the sulfated fucan sulfatase of the present invention used in the reaction, the composition of the reaction mixture, the reaction time and the like. The sulfated fucan oligosaccharide of the present invention having more homogeneous molecular weight or charge density distribution can be prepared by subjecting the sulfated fucan oligosaccharide of the present invention obtained as described above to molecular weight fractionation or fractionation using an anion exchange column. A conventional method of molecular weight fractionation (e.g., gel filtration or ultrafiltration) may be used. Optionally, the smaller molecules may be further purified using ion exchange resin treatment, active carbon treatment or the like, or they may be desalted, sterilized or lyophilized.

The sulfated fucan oligosaccharides of the present invention have sulfate groups within the molecules, which groups form salts with various bases. The sulfated fucan oligosaccharide of the present invention is stable when it is in a form of salt. It is usually provided in a form of sodium and/or potassium and/or calcium salt. The sulfated fucan oligosaccharide of the present invention in a free form can be derived from a salt thereof by utilizing cation exchange resin such as Dowex 50W. Optionally, it can be subjected to conventional salt-exchange to convert it into any one of various desirable salts.

Pharmaceutically acceptable salts can be used as the salts of the sulfated fucan oligosaccharide of the present invention. Examples of the salts include salts with alkaline metals (e.g., sodium and potassium) and alkaline earth metals (e.g., calcium, magnesium and zinc) as well as ammonium salts.

The endo-type $\alpha$-L-fucosidase and the sulfated fucan sulfatase of the present invention can be used for structural analysis of a sulfated fucan because they convert a sulfated fucan into smaller molecules. Additionally, the sulfated fucan oligosaccharide of the present invention can be used as a reagent for glycotechnology. For example, a substance that is very useful as a reagent for glycotechnology (e.g., that can be used as a fluorescence labeled standard for sulfated fucan oligosaccharides) can be provided by subjecting the oligosaccharide to amination with 2-aminopyridine (PA-labeling) according to the method as described in JP-B 5-65108 to prepare a PA-labeled oligosaccharide. Furthermore, the sulfated fucan oligosaccharide of the present invention can be utilized as an antigen to produce an antibody that recognizes the sulfated fucan oligosaccharide of the present invention according to a known method. An antibody that recognizes the sulfated fucan oligosaccharide of the present invention can be utilized to analyze the structure of a sulfated polysaccharide and, therefore, is very useful.

Examples

The following examples further illustrate the present invention in detail but are not to be construed to limit the scope thereof.

Referential Example 1: Preparation of sea cucumber-derived sulfated polysaccharide fraction

The body wall portions of 35 Kg of commercially available sea cucumbers were finely cut, and treated with four volumes of acetone in a homogenizer at 8000 rpm for 5 minutes. The homogenate was then filtered through a filter paper to obtain a residue. The residue was treated with four volumes of acetone in a homogenizer at 8000 rpm for 5 minutes. The homogenate was then filtered through a filter paper to obtain a residue. The thus obtained residue was dried by aspiration to obtain 1132 g of an acetone treatment preparation of sea cucumber body wall.
400 g of the acetone treatment preparation of sea cucumber body wall was suspended in 10 L of 30 mM imidazole-hydrochloride buffer (pH 6.5) containing 100 mM sodium chloride and 10 mM calcium chloride, 2 g of Actinase E was added thereto, and the mixture was treated at 45°C for 3 hours and then at 95°C for 2 hours. The treated mixture was filtered through a stainless steel sieve (pore size: 106 $\mu$m), 20 g of active carbon was added to the resulting filtrate, and the mixture was stirred at room temperature for 30 minutes. The mixture was then centrifuged to obtain a supernatant, and ethanol was added to the supernatant at a final concentration of 10%. The mixture was concentrated to 2 L using an ultrafiltration device equipped with hollow fibers with exclusion molecular weight of 100,000. The concentrate was centrifuged to remove insoluble substances. The resulting supernatant was subjected to solvent exchange for 100 mM sodium chloride containing 10% ethanol using an ultrafiltration device. The solution was cooled to 4°C or below, and the pH was adjusted to 2.0 with hydrochloric acid. The formed precipitate was removed by centrifugation to obtain a supernatant. The pH of the supernatant was adjusted to 8.0 with sodium hydroxide. The supernatant was subjected to solvent exchange for 20 mM sodium chloride using an ultrafiltration device. Insoluble substances were removed from the solution by centrifugation. The supernatant was then lyophilized to obtain 37 g of a dried product of sea cucumber-derived sulfated polysaccharide fraction.

Referential Example 2: Preparation of sulfated fucan fraction

7 g of the dried product of sea cucumber-derived sulfated polysaccharide fraction as described in Reference Example 1 was dissolved in 700 ml of 20 mM imidazole-hydrochloride buffer (pH 7.0) containing 50 mM sodium chloride and 10% ethanol, and insoluble substances were removed by centrifugation. The resulting supernatant was loaded onto a 5-L DEAE-Cellulofine A-800 column equilibrated with the same buffer. After washing with the same buffer, elution was carried out with a gradient of 50 mM to 2.05 M sodium chloride to collect fractions each containing 500 ml of the eluate. The total sugar content and the uronic acid content of each fraction were measured according to the phenol-sulfuric acid method and the carbazole-sulfuric acid method, respectively. Fractions eluted with 0.8 to 1.5 M sodium chloride (fraction nos. 45-57) were collected to obtain a sulfated fucan fraction.

Referential Example 3: Method for measuring activity of endo-type $\alpha$-L-fucosidase

15 $\mu$l of a 1% solution of the sulfated fucan fraction, 75 $\mu$l of 50 mM sodium phosphate buffer (pH 8.5), 9 $\mu$l of 4 M sodium chloride, 41 $\mu$l of water and 10 $\mu$l of a solution of the endo-type $\alpha$-L-fucosidase of the present invention were mixed together. After reacting at 37°C for 1 hour, the reaction mixture was treated at 100°C for 10 minutes. 100 $\mu$l of a supernatant obtained by centrifuging the mixture was analyzed using HPLC to determine the degree of conversion into smaller molecules. As controls, a reaction mixture obtained by a reaction in which the buffer used for dissolving the endo-type $\alpha$-L-fucosidase of the present invention was used in place of the enzyme solution and a reaction mixture obtained by a reaction in which water was used in place of the sulfated fucan fraction were analyzed using HPLC in a similar manner.

One unit of an endo-type α-L-fucosidase activity is defined as an amount of an enzyme that cleaves one μmol of sulfated fucan fucosyl bonds in one minute in the above-mentioned reaction system. The endo-type α-L-fucosidase activity was calculated according to the following equation:

$$\{(15\times1000\times1/100)/MG\}\times\{(MG/M)-1\}\times\{1/(60\times0.01)\}=U/ml$$

15x1000x1/100: the sulfated fucan fraction added to the reaction system (μg);
MG: the average molecular weight of the sulfated fucan as a substrate;
M: the average molecular weight of the reaction product;
(MG/M)-1: the number of sites cleaved by the enzyme in one molecule of the sulfated fucan;
60: the reaction time (minutes); and
0.01: the volume of the enzyme solution (ml).

The HPLC was carried out as follows.

Instrument: L-6200 (Hitachi);
Column: OHpak SB-806HQ (8 x 300 mm; Showa Denko);
Eluent: 50 mM sodium chloride containing 5 mM sodium azide;
Detection: differential refractive index detector (Shodex RI-71, Showa Denko);
Flow rate: 1 ml/minute; and
Column temperature: 25°C.

The following procedure was carried out in order to determine the average molecular weight of the reaction product. Commercially available pullulan (STANDARD P-82, Showa Denko) of which the molecular weight was known was analyzed under the same conditions as those for the above-mentioned HPLC analysis. The relationship between the molecular weight of pullulan and the retention time was expressed as a curve, which was used as a standard curve for determining the molecular weight of the reaction product. The amount of protein was determined by measuring the absorbance of the enzyme solution at 280 nm. The calculation was carried out defining the absorbance of a solution containing a protein at a concentration of 1 mg/ml as 1.0.

Referential Example 4: Method for measuring activity of sulfated fucan sulfatase

15 μl of a 1% solution of the sulfated fucan fraction, 75 μl of 50 mM imidazole-hydrochloride buffer (pH 7.0), 6 μl of 4 M sodium chloride, 3 μl of 1 M calcium chloride, 31 μl of water and 20 μl of a solution of the sulfated fucan sulfatase of the present invention were mixed together. After reacting at 37°C for 1 hour, the reaction mixture was treated at 100°C for 10 minutes. 100 μl of a supernatant obtained by centrifuging the mixture was analyzed using HPLC to determine the amount of generated sulfuric acid. As controls, a reaction mixture obtained by a reaction in which the buffer used for dissolving the sulfated fucan sulfatase of the present invention was used in place of the enzyme solution and a reaction mixture obtained by a reaction in which water was used in place of the sulfated fucan fraction were analyzed using HPLC in a similar manner.
One unit of a sulfated fucan sulfatase activity is defined as an amount of an enzyme that generates one μmol of sulfuric acid in one minute in the above-mentioned reaction system. The sulfated fucan sulfatase activity was calculated according to the following equation:

$$S/(60\times0.02)=U/ml$$

S: sulfuric acid generated by the reaction (μmole);
60: the reaction time (minutes); and
0.02: the volume of the enzyme solution (ml).

The HPLC was carried out as follows.

Instrument: L-6200 (Hitachi);
Column: OHpak SB-804HQ (8 x 300 mm; Showa Denko);
Eluent: 50 mM sodium chloride containing 5 mM sodium azide;
Detection: differential refractive index detector (Shodex RI-71, Showa Denko);

Flow rate: 1 ml/minute; and
Column temperature: 35°C.

1 mM or 0.5 mM sodium sulfate was analyzed using HPLC under the same conditions in order to measure the amount of sulfuric acid released as a result of the reaction. The relationship between the sodium sulfate concentration and the peak height was expressed as a curve, which was used as a standard curve for measuring the amount of released sulfuric acid.

Example 1: Preparation of endo-type α-L-fucosidase (1)

*Fucoidanobacter marinus* SI-0098 was inoculated into 5 ml of a medium consisting of artificial seawater (Jamarine Laboratory) containing the sea cucumber-derived sulfated polysaccharide fraction prepared as described in Referential Example 1 and peptone at concentrations of 0.3% and 1%, respectively, (pH 8.2) which had been autoclaved at 120°C for 20 minutes, and cultured at 25°C for two days. 150 μl of the culture was inoculated into 100 ml of a medium containing the same components, and cultured at 25°C for three days. Seven 2-L Erlenmeyer flasks each containing 600 ml of a medium containing the same components and an antifoaming agent (KM70, Shin-Etsu Chemical) at a concentration of 0.01% were autoclaved at 120°C for 20 minutes. One ml of the culture was inoculated into each medium and cultured at 25°C for three days. After cultivation, the culture was centrifuged to obtain cells and a culture supernatant. The same cultivation procedure was repeated to obtain about 15 g of wet cells from 12.6 L of the culture.

The cells were suspended in 200 ml of 10 mM imidazole-hydrochloride buffer, (pH 7.5) containing 100 mM sodium chloride and 5 mM β-mercaptoethanol, disrupted by sonication and centrifuged to obtain a supernatant. The supernatant was adequately dialyzed against the same buffer and centrifuged to obtain a supernatant.

The supernatant was loaded onto a 500-ml DEAE-Cellulofine A-800 column equilibrated with the same buffer. After washing with the same buffer, elution was carried out with a gradient of 100 to 500 mM sodium chloride to collect fractions each containing 45 ml of the eluate. The endo-type α-L-fucosidase activities were measured for the respective fractions and an active fraction was collected.

The active fraction was adequately dialyzed against 10 mM imidazole-hydrochloride buffer (pH 7.5) containing 50 mM sodium chloride and 5 mM β-mercaptoethanol for solvent exchange. The enzyme solution was loaded onto a 150-ml DEAE-Cellulofine A-800 column equilibrated with the same buffer. After washing with the same buffer, elution was carried out with a gradient of 50 to 400 mM sodium chloride to collect fractions each containing 20 ml of the eluate. The endo-type α-L-fucosidase activities were measured for the respective fractions and an active fraction was collected.

The active fraction was adequately dialyzed against 10 mM imidazole-hydrochloride buffer (pH 7.5) containing 50 mM sodium chloride and 5 mM β-mercaptoethanol for solvent exchange. The enzyme solution was loaded onto a 80-ml Sulfated Cellulofine column equilibrated with the same buffer. After washing with the same buffer, elution was carried out with a gradient of 50 to 1500 mM sodium chloride to collect fractions each containing 5 ml of the eluate. The endo-type α-L-fucosidase activities were measured for the respective fractions and an active fraction was collected.

The active fraction was adequately dialyzed against 10 mM imidazole-hydrochloride buffer (pH 7.5) containing 100 mM sodium chloride and 5 mM β-mercaptoethanol followed by 40 mM potassium phosphate buffer containing 150 mM sodium chloride and 5 mM β-mercaptoethanol for solvent exchange. The enzyme solution was loaded onto a 40-ml Hydroxyapatite column (Wako Pure Chemical Industries) equilibrated with the same buffer. After washing with the same buffer, elution was carried out with a gradient of 40 to 250 mM potassium phosphate to collect fractions each containing 6 ml of the eluate. The endo-type α-L-fucosidase activities were measured for the respective fractions and an active fraction was collected.

The active fraction was adequately dialyzed against 10 mM imidazole-hydrochloride buffer (pH 7.5) containing 100 mM sodium chloride and 5 mM p-mercaptoethanol for solvent exchange. The enzyme solution was loaded onto a 1520-ml Sephacryl S-200 column (4.4 x 100 cm, Pharmacia) equilibrated with the same buffer. Elution was carried out with the same buffer to collect fractions each containing 13.5 ml of the eluate. The endo-type α-L-fucosidase activities were measured for the respective fractions.

Thus, a purified preparation of the endo-type α-L-fucosidase of the present invention was obtained. The molecular weight of the endo-type α-L-fucosidase of the present invention was determined to be about 32,000 (distribution: 20,000 to 40,000, based on the position of elution upon gel filtration on Sephacryl S-200) or 42,000 (distribution: 30,000 to 50,000, based on the mobility upon electrophoresis).

The purification procedure is summarized in Table 1 below.

Table 1

| Step | Total protein (mg) | Total activity (mU) | Specific activity (mU/mg) | Yield (%) |
|---|---|---|---|---|
| Extract | 5,520 | 20,400 | 3.700 | 100 |

Table 1   (continued)

| Step | Total protein (mg) | Total activity (mU) | Specific activity (mU/mg) | Yield (%) |
|---|---|---|---|---|
| DEAE-Cellulofine | 193 | 10,800 | 56.0 | 52.9 |
| DEAE-Cellulofine | 140 | 6,700 | 41.7 | 32.8 |
| Sulfated Cellulofine | 4.58 | 1,860 | 406 | 9.12 |
| Hydroxyapatite | 2.12 | 2,440 | 1,150 | 12.0 |
| Sephacryl S-200 | 1.40 | 1,220 | 871 | 5.98 |

Example 2: Preparation of endo-type $\alpha$-L-fucosidase (2)

*Fucoidanobacter marinus* SI-0098 was cultured as described in Example 1 to obtain about 6 g of wet cells from 4.2 L of the culture.

The cells were suspended in 50 ml of 10 mM sodium phosphate buffer (pH 7.5) containing 50 mM sodium chloride, disrupted by sonication and centrifuged to obtain a supernatant. The supernatant was adequately dialyzed against the same buffer and centrifuged to obtain a supernatant.

The supernatant was loaded onto a 300-ml DEAE-Cellulofine A-800 column equilibrated with the same buffer. After washing with the same buffer, elution was carried out with a gradient of 50 to 500 mM sodium chloride to collect fractions each containing 25 ml of the eluate. The endo-type $\alpha$-L-fucosidase activities were measured for the respective fractions and an active fraction was collected.

The active fraction was adequately dialyzed against 10 mM sodium phosphate buffer (pH 7.5) containing 100 mM sodium chloride for solvent exchange. The enzyme solution was loaded onto a 50-ml DEAE-Cellulofine A-800 column equilibrated with the same buffer. After washing with the same buffer, elution was carried out with a gradient of 100 to 400 mM sodium chloride to collect fractions each containing 10 ml of the eluate. The endo-type $\alpha$-L-fucosidase activities were measured for the respective fractions and an active fraction was collected.

The active fraction was adequately dialyzed against 50 mM potassium phosphate buffer (pH 7.5) containing 50 mM sodium chloride for solvent exchange. The enzyme solution was loaded onto a 20-ml Hydroxyapatite column equilibrated with the same buffer. After washing with the same buffer, elution was carried out with a gradient of 50 to 200 mM potassium phosphate to collect fractions each containing 10 ml of the eluate. The endo-type $\alpha$-L-fucosidase activities were measured for the respective fractions and an active fraction was collected.

The active fraction was loaded onto a 1520-ml Sephacryl S-200 column (4.4 x 100 cm, Pharmacia) equilibrated with 10 mM imidazole-hydrochloride buffer (pH 7.5) containing 100 mM sodium chloride and 5 mM sodium azide. Elution was carried out with the same buffer to collect fractions each containing 13.5 ml of the eluate. The endo-type $\alpha$-L-fucosidase activities were measured for the respective fractions.

Thus, a purified preparation of the endo-type $\alpha$-L-fucosidase of the present invention was obtained.

The purification procedure is summarized in Table 2 below.

Table 2

| Step | Total protein (mg) | Total activity (mU) | Specific activity (mU/mg) | Yield (%) |
|---|---|---|---|---|
| Extract | 2,067 | 8,010 | 3.88 | 100 |
| Dialysis | 1,923 | 6,980 | 3.63 | 87.1 |
| DEAE-Cellulofine | 53.3 | 6,590 | 124 | 82.3 |
| DEAE-Cellulofine | 29.2 | 6,500 | 223 | 81.1 |
| Hydroxyapatite | 3.91 | 3,220 | 824 | 40.2 |
| Sephacryl S-200 | 1.19 | 2,280 | 1,920 | 28.4 |

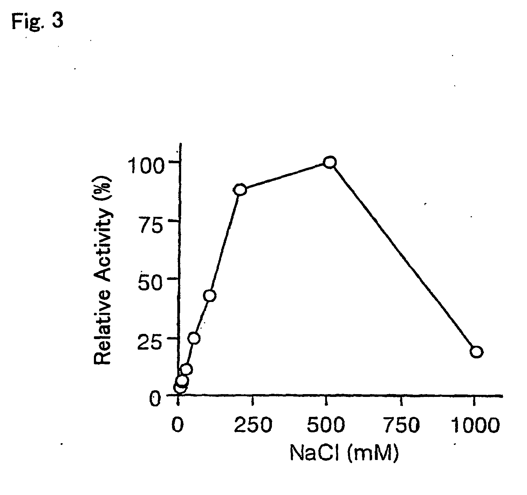

The optimal pH, temperature and salt concentration were studied for the purified preparation of the endo-type $\alpha$-L-fucosidase of the present invention. The results are shown in Figures 1, 2 and 3, respectively.

Example 3: Preparation of sulfated fucan oligosaccharides using endo-type $\alpha$-L-fucosidase

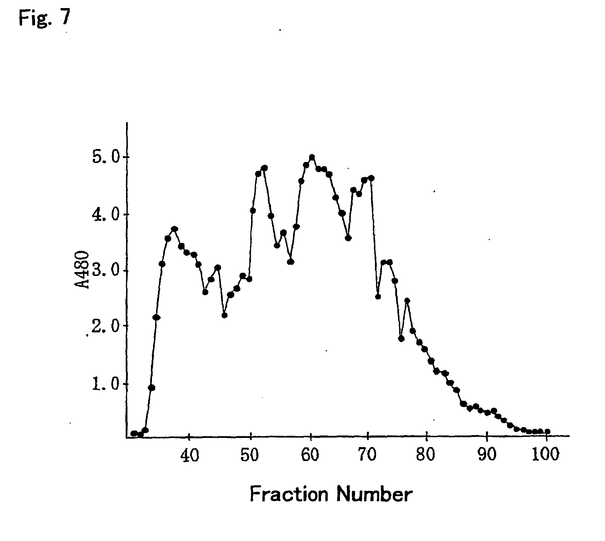

1.5 g of the sulfated fucan fraction as described in Referential Example 2 was dissolved in 135 ml of 50 mM imidazole-hydrochloride buffer (pH 8.2) containing 250 mM sodium chloride. 144 mU (15 ml) of the endo-type $\alpha$-L-fucosidase of the present invention was added thereto. The mixture was stirred at 30°C for four days. Three portions each consisting of 50 ml of the mixture were subjected to gel filtration on a 1100-ml Cellulofine GCL-1000 (4 x 87 cm, Seikagaku Corporation) for molecular weight fractionation. The total sugar content of each elute fraction was measured according to the phenol-sulfuric acid method. As a result, the distribution as shown in Figure 7 was observed. In Figure 7, the longitudinal axis represents absorbance at 480 nm observed upon color development according to the phenol-sulfuric acid method, and the horizontal axis represents fraction numbers. Thus, the sulfated fucan oligosaccharides of the present invention were eluted around fraction nos. 50-75 with distribution of molecular weight around 40,000. The oligosaccharides were subjected to analyses of sugar compositions and saccharides at the reducing ends. As a result, fucose was observed for substantially all cases.

The sulfated fucan oligosaccharides of the present invention having varying molecular weights could be prepared by allowing the endo-type $\alpha$-L-fucosidase of the present invention to act on the sea cucumber-derived sulfated fucan fraction.

Example 4: Sulfated fucan sulfatase

It was impossible to convert the sea cucumber-derived sulfated fucan into molecules smaller than the molecular weight distribution as described in Example 3 when the endo-type $\alpha$-L-fucosidase of the present invention prepared as described in Example 1 or 2 was used alone. Then, a fraction whose coexistence with the endo-type $\alpha$-L-fucosidase of the present invention results in conversion of the sea cucumber-derived sulfated fucan fraction into smaller molecules was searched. As a result, an activity that activates the conversion of the sulfated fucan into smaller molecules by the endo-type $\alpha$-L-fucosidase of the present invention was found in a fraction eluted from Sulfated Cellulofine with about 600 mM sodium chloride in Example 1. Then, the fraction was allowed to act on the sea cucumber-derived sulfated fucan and substances generated in the reaction mixture were examined. First, the reaction mixture was analyzed according to the method as described in Referential Example 4 using HPLC, and generation of sulfuric acid was observed. Next, the total sugar content was measured according to the phenol-sulfuric acid method for each fraction eluted upon gel filtration of the reaction mixture on Cellulofine GCL-25 column (4 x 90 cm) in order to examine whether or not a smaller-molecule sugar-containing component was generated. As a result, generation of a smaller-molecule sugar-containing component was not found. Based on the above, it was strongly suggested that the enzyme that activates the conversion the sulfated fucan into smaller molecules by the endo-type $\alpha$-L-fucosidase of the present invention was a sulfated fucan sulfatase. Then, a sulfated fucan sulfatase whose activity is detected according to the method as described in Referential Example 4 was purified.

Specifically, an active fraction of the sulfated fucan sulfatase of the present invention which were eluted from Sulfated Cellulofine with around 600 mM sodium chloride in Example 1 was collected, and loaded onto a 1520-ml Sephacryl S-200 column equilibrated with 10 mM imidazole-hydrochloride buffer containing 100 mM sodium chloride, 5 mM sodium azide and 5 mM β-mercaptoethanol. Elution was carried out with the same buffer to collect fractions each containing 13.5 ml of the eluate. The molecular weight of the sulfated fucan sulfatase of the present invention was determined to be about 84,000 (distribution: 70,000 to 100,000) based on the position of elution.

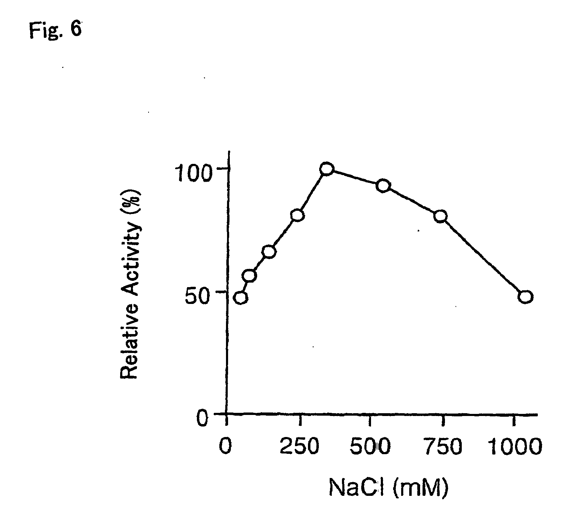

The optimal pH, temperature and salt concentration were studied for the purified preparation of the sulfated fucan sulfatase of the present invention. The results are shown in Figures 4, 5 and 6, respectively.

Example 5: Effect of sodium salt concentration

The relationship between the relative activity and the concentration of sodium chloride contained in the reaction system for the endo-type $\alpha$-L-fucosidase or the sulfated fucan sulfatase of the present invention was studied. As a result, it was found that the endo-type $\alpha$-L-fucosidase and the sulfated fucan sulfatase of the present invention are activated by sodium chloride.

Example 6: Effect of calcium salt concentration

The relationship between the relative activity and the concentration of calcium chloride contained in the reaction system for the sulfated fucan sulfatase of the present invention was studied. As a result, it was found that the sulfated fucan sulfatase of the present invention is activated by calcium chloride. Activation was similarly observed using calcium acetate.

Example 7: Preparation of sulfated fucan oligosaccharides using endo-type $\alpha$-L-fucosidase and sulfated fucan sulfatase

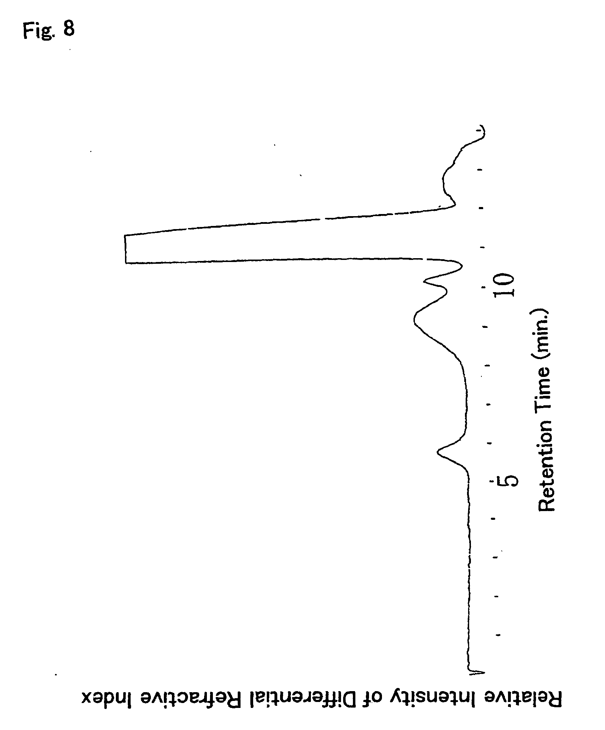

150 μl of 50 mM imidazole-hydrochloride buffer (pH 7.0), 14 μl of 4 M sodium chloride, 6 μl of 1 M calcium chloride and 64 μl of water were mixed with 30 μl of a 1% aqueous solution of the sea cucumber-derived sulfated fucan fraction as described in Referential Example 2. 424 μU (16 μl) of the endo-type $\alpha$-L-fucosidase of the present invention and 226 μU (20 μl) of the sulfated fucan sulfatase of the present invention were added thereto. The mixture was reacted at 30°C for two days. 100 μl of the reaction mixture was analyzed according to the method as described in Referential Example 4 using HPLC. The results are shown in Figure 8. In Figure 8, the longitudinal axis represents the relative intensity of differential refractive index, and the horizontal axis represents the retention time. The generated sulfated fucan oligosaccharides of the present invention were distributed at positions of 7 to 10 minutes corresponding to a molecular weight of around 9,000. Thus, oligosaccharides which are clearly smaller than the oligosaccharides as described in Example 3 could be obtained. The oligosaccharides were subjected to analyses of sugar compositions and saccharides at the reducing ends. As a result, fucose was observed for substantially all cases.

Thus, it was found that the sulfated fucan oligosaccharides of the present invention having varying molecular weights can be prepared by allowing the endo-type $\alpha$-L-fucosidase and the sulfated fucan sulfatase of the present invention to act on the sea cucumber-derived sulfated fucan fraction.

Industrial Applicability

The present invention provides a novel endo-type $\alpha$-L-fucosidase and a novel sulfated fucan sulfatase which can be used for structural analysis of a sulfated fucan derived from a sea cucumber or reproducible production of smaller molecules from a sulfated fucan derived from a sea cucumber. The present invention also provides methods of producing the enzymes. Furthermore, sea cucumber-derived sulfated fucan oligosaccharides having varying molecular weights, which are useful as reagents for glycotechnology, are provided by using the enzymes. In addition, additives for efficient uses of the enzymes are provided.

**Claims**

1. An endo-type α-L-fucosidase having the following physical and chemical properties:

   (I) acting on a sulfated fucan derived from a sea cucumber to hydrolyze an α-L-fucosyl bond and convert the sulfated fucan into smaller molecules;
   (II) having an optimal pH of about 7 to 9; and
   (III) having an optimal temperature of about 25 to 45°C.

2. A method for producing the endo-type α-L-fucosidase defined by claim 1, the method comprising culturing a bacterium of the genus *Fucoidanobacter* capable of producing the endo-type α-L-fucosidase defined by claim 1, and collecting the enzyme from the culture.

3. A sulfated fucan sulfatase having the following physical and chemical properties:

(I) acting on a sulfated fucan derived from a sea cucumber to hydrolyze a sulfate ester bond, release sulfuric acid, and promote conversion of the sulfated fucan derived from a sea cucumber into smaller molecules in the presence of the endo-type $\alpha$-L-fucosidase defined by claim 1 as compared with conversion achieved by allowing the endo-type $\alpha$-L-fucosidase defined by claim 1 to act alone;
(II) having an optimal pH of about 6 to 8; and
(III) having an optimal temperature of about 20 to 45°C.

4. A method for producing the sulfated fucan sulfatase defined by claim 3, the method comprising culturing a bacterium of the genus *Fucoidanobacter* capable of producing the sulfated fucan sulfatase defined by claim 3, and collecting the enzyme from the culture.

5. A method for producing a sulfated fucan oligosaccharide, the method comprising allowing the endo-type $\alpha$-L-fucosidase defined by claim 1 to act on a sulfated fucan, and obtaining a sulfated fucan oligosaccharide from the reaction.

6. The method according to claim 5, wherein the endo-type $\alpha$-L-fucosidase is allowed to act in the presence of sodium chloride.

7. A sulfated fucan oligosaccharide which is obtainable by the method defined by claim 5.

8. A method for producing a sulfated fucan oligosaccharide, the method comprising allowing the endo-type $\alpha$-L-fucosidase defined by claim 1 and the sulfated fucan sulfatase defined by claim 3 to act on a sulfated fucan, and obtaining a sulfated fucan oligosaccharide from the reaction.

9. The method according to claim 8, wherein the endo-type $\alpha$-L-fucosidase and the sulfated fucan sulfatase are allowed to act in the presence of sodium chloride and/or a calcium ion.

10. A sulfated fucan oligosaccharide which is obtainable by the method defined by claim 8.

Fig. 1

Fig. 2

Fig. 3

Fig. 4

Fig. 5

Fig. 6

Fig. 7

Fig. 8

## INTERNATIONAL SEARCH REPORT

| International application No. |
|---|
| PCT/JP03/07838 |

| A. CLASSIFICATION OF SUBJECT MATTER |
|---|
| Int.Cl$^7$  C12N9/44, C12N9/16, C12N19/04, C08B37/00//C12N15/12 |

According to International Patent Classification (IPC) or to both national classification and IPC

| B. FIELDS SEARCHED |
|---|
| Minimum documentation searched (classification system followed by classification symbols) |
| Int.Cl$^7$  C12N9/44, C12N9/16, C12N19/04, C08B37/00, C12N15/12 |

Documentation searched other than minimum documentation to the extent that such documents are included in the fields searched

Electronic data base consulted during the international search (name of data base and, where practicable, search terms used)
JSTPlus(JOIS), BIOSIS(DIALOG), WPI(DIALOG)

### C. DOCUMENTS CONSIDERED TO BE RELEVANT

| Category* | Citation of document, with indication, where appropriate, of the relevant passages | Relevant to claim No. |
|---|---|---|
| X<br>A | Pereira M.S. et al., Structure and anticoagulant activity of sulfated fucans. Comparison between the regular, repetitive, and linear fucans from echinoderms with the more heterogeneous and branched polymers from brown algae, J.Biol.Chem., 1999, Vol.274, No.12, pages 7656-67 | 7,10<br>1-6,8-9 |
| X<br>A | WO 01/081560 A1 (Takara Shuzo Co., Ltd.), 01 November, 2001 (01.11.01), Full text<br>& EP 1277834 A1 | 7,10<br>1-6,8-9 |
| X<br>A | WO 96/34004 A1 (Takara Shuzo Co., Ltd.), 31 October, 1996 (31.10.96), Full text<br>& EP 870771 A1        & US 6277616 B1<br>& US 6379947 B2 | 7,10<br>1-6,8-9 |

| ☒ Further documents are listed in the continuation of Box C. | ☐ See patent family annex. |
|---|---|

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>29 August, 2003 (29.08.03) | Date of mailing of the international search report<br>09 September, 2003 (09.09.03) |
| Name and mailing address of the ISA/<br>Japanese Patent Office | Authorized officer |
| Facsimile No. | Telephone No. |

Form PCT/ISA/210 (second sheet) (July 1998)

**EP 1 514 923 A1**

## INTERNATIONAL SEARCH REPORT

| International application No. |
|---|
| PCT/JP03/07838 |

C (Continuation). DOCUMENTS CONSIDERED TO BE RELEVANT

| Category* | Citation of document, with indication, where appropriate, of the relevant passages | Relevant to claim No. |
|---|---|---|
| P,X | Takeshi SAKAI et al., "Namako Yurai Ryusan-ka $\alpha$-L-fucan o Bunkai suru Kaiyo Saikin Yurai endo-$\alpha$-L-fucosidase oyobi sulfatase", Dai 23 kai The Japanese Society of Carbohydrate Research Nenkai Yoshishu, 2002 July, page 136, PII-55 | 1-10 |

Form PCT/ISA/210 (continuation of second sheet) (July 1998)

24